# EUROPÄISCHE PATENTANMELDUNG

(11) **EP 1 147 769 A2**
(43) Veröffentlichungstag der Anmeldung: **24.10.2001**
(21) Anmeldenummer: 01108445.6
(22) Anmeldetag: 04.04.2001
(51) Int. Cl.: A61K 31/202, A61K 31/232, A61P 13/02

(54) **Verwendung von mehrfach ungesättigten Fettsäuren, insbesondere Omega-3-Fettsäuren, zur Behandlung von Enuresis und Inkontinenz**

(30) Priorität: 18.04.2000 DE 10019052
(71) Anmelder: Bitec GmbH, 66450 Bexbach (DE); NeuroNet Institut für Hirnforschung & angewandte Technologie G.m.b.H., 66606 St. Wendel (DE)
(72) Erfinder: Brill, Klaus, Dr. med., 66606 St. Wendel (DE); Weiler, Elmar W.J., Dr., 66620 Nonnweiler (DE)
(74) Vertreter: Bernhardt, Winfrid, Dr.-Ing.

(57) **Zusammenfassung**

Mehrfach ungesättigte Fettsäuren und ihre Verbindungen können zur erfolgreichen Behandlung von Enuresis und Inkontinenz verwendet werden.

## Beschreibung

Die Erfindung betrifft eine Verwendung von mehrfach ungesättigten Fettsäuren, insbesondere Omega-3-Fettsäuren.

Von den mehrfach ungesättigten Fettsäuren sind verschiedene gesundheitsfördernde Wirkungen bekannt. So wird ein im Handel befindliches Arzneimittel mit Wirkstoff Lachsöl-Konzentrat für folgende Anwendungsgebiete empfohlen: Günstiger Einfluß auf das Blutfettsystem (Cholesterin, Triglyceride, LDL und HDL), auf die Gerinnungseigenschaften und die Fließfähigkeit des Blutes, auf das Blutdruckverhalten und auf die Leistung des Gehirns, z.B. die Konzentrations- und Lernfähigkeit.
In DMW (Deutsche medizinische Wochenschrift) 1990, Seiten 224 bis 231 wird über Behandlungen der Atherosklerose und chronisch entzündlicher Erkrankungen sowie über weitere Untersuchungen berichtet.

Überraschend wurde jetzt gefunden, daß die mehrfach ungesättigten Fettsäuren und ihre Verbindungen zur erfolgreichen Behandlung von Enuresis und Inkontinenz verwendet werden können.

Eingesetzt wurde das oben bereits erwähnte Arzneimittel. In diesem enthält eine Kapsel 500 mg Lachsöl-Konzentrat (18% Eicosapentaensäure, 12% Docosahexaensäure, Omega-3-Fettsäuren insgesamt mindestens 35%, Tocopherol (Vitamin E) 1 mg); sonstige Bestandteile: Tocopherolacetat, Gelatine, Glycerol.

Eingesetzt wurde ferner ein Nahrungsergänzungspräparat. Von diesem enthielt eine Kapsel 500 mg Fettsäureethylester, davon mindestens 90% Omega-3-Fettsäuren (Eicosapentaensäure und Docosahexaensäure zusammen 80%, Stearidonsäure und Heneicosapentaensäure zusammen 10%), Rest hauptsächlich andere ungesättigte Fettsäuren.

Die verabreichte Dosis betrug jeweils 1 bis 2 Kapseln pro Tag.

Gegen Enuresis (Bettnässen) behandelt wurden bisher acht Probanden männlichen Geschlechts zwischen 5 und 16 Jahren.
Der Erfolg stellte sich nach 1 Tag bis 2 Wochen ein und war dauerhaft. Zur Probe wurde in zwei Fällen das Präparat abgesetzt und später wieder verabreicht. Sofort nach dem Absetzen des Präparats stellte sich das Bettnässen wieder ein, und sofort nach der Wiedereinnahme war es erneut beseitigt.
Im Falle einer zweijährigen Einnahme blieb nach dem Absetzen des Präparats der Erfolg bisher erhalten.
In zwei der behandelten Fälle blieb ein Erfolg aus, in zwei anderen Fällen bestand er nicht in einer vollständigen Beseitigung, aber einer wesentlichen Besserung.

Überraschend ist auch die geringe Dosis, die sich bereits als wirksam erwiesen hat. In den beiden Fällen des Mißerfolgs war das erstere Präparat gegeben worden; das zweite Präparat mit der gegenüber dem ersten 2,5-fachen Wirkstoffmenge läßt eine höhere Ansprechquote erwarten.

Von dem Bettnässen, Enuresis, als der unwillkürlichen Blasenentleerung, wird die Inkontinenz unterschieden als das Unvermögen, den Harn willkürlich zurückzuhalten.
Die Mechanismen sind nicht gleich, überschneiden sich aber.
Wie sich an bisher zwei Probanden gezeigt hat, üben die mehrfach ungesättigten Fettsäuren und ihre Verbindungen auch bei der Inkontinenz eine Besserungswirkung aus.

Bei den Probanden eingesetzt wurde das oben angegebene Nahrungsergänzungspräparat. Die verabreichte Dosis betrug jeweils 1 bis 3 Kapseln pro Tag.
In Betracht kommt ferner das oben bereits erwähnte Arzneimittel.

Allgemein werden für die erfindungsgemäßen Verwendungen geeignete Präparate in der Regel, wie Präparate ungesättigter Fettsäuren bisher, aus Fischöl mit hohen Anteilen an Omega-3-Fettsäuren hergestellt sein.

Im Falle eines bloßen Fischöl-Konzentrats liegen die Fettsäuren dann in der Verbindung der Triglyceride vor.
Höher angereicherte Produkte bestehen, je nach angewandtem Verfahren, aus den Fettsäuren selbst oder Estern oder anderen Verbindungen der Fettsäuren. Vor allem in Betracht stehen die Stearidonsäure 18:4, die Eicosatetraensäure 20:4 (Omega 3), die Eicosapentaensäure 20:5, die Docosapentaensäure 22:5 und die Docosahexaensäure 22:6 sowie Verbindungen dieser Säuren.

## Patentansprüche

1. Verwendung mindestens einer mehrfach ungesättigten Fettsäure und/oder mindestens einer Verbindung einer solchen Fettsäure zur therapeutischen Behandlung von Enuresis und Inkontinenz.

2. Verwendung nach Anspruch 1 mindestens einer Omega-3-Fettsäure und/oder mindestens einer Verbindung einer Omega-3-Fettsäure.

3. Verwendung nach Anspruch 2 von Stearidonsäure, Eicosatetraensäure, Eicosapentaensäure, Docosapentaensäure und/oder Docosahexaensäure und/oder mindestens einer Verbindung mindestens einer dieser Säuren.

4. Verwendung nach einem der Ansprüche 1 bis 3 von Äthylester(n) als der der genannten Verbindung(en).
